(19) **Europäisches Patentamt / European Patent Office / Office européen des brevets**

(11) **EP 2 279 739 B2**

(12) **NEW EUROPEAN PATENT SPECIFICATION**

After opposition procedure

(45) Date of publication and mention of the opposition decision:
**28.02.2018   Bulletin 2018/09**

(45) Mention of the grant of the patent:
**11.06.2014   Bulletin 2014/24**

(21) Application number: **09754685.7**

(22) Date of filing: **26.05.2009**

(51) Int Cl.:
*A61K 31/4465* (2006.01)    *A61K 9/70* (2006.01)
*A61K 47/32* (2006.01)    *A61P 25/28* (2006.01)
*A61P 43/00* (2006.01)    *B65D 81/24* (2006.01)
*B65D 81/26* (2006.01)

(86) International application number:
**PCT/JP2009/059588**

(87) International publication number:
**WO 2009/145177 (03.12.2009 Gazette 2009/49)**

(54) **DONEPEZIL-CONTAINING PATCH PREPARATION AND PACKAGING THEREOF**

DONEPEZILHALTIGES PFLASTERPRÄPARAT UND DESSEN VERPACKUNG

PRÉPARATION DE PATCH CONTENANT DU DONÉZÉPIL ET CONDITIONNEMENT

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority:  **30.05.2008   JP 2008143438**

(43) Date of publication of application:
**02.02.2011   Bulletin 2011/05**

(73) Proprietor: **Nitto Denko Corporation**
**Ibaraki-shi**
**Osaka 567-8680 (JP)**

(72) Inventors:
• **SEKIYA, Junichi**
**Ibaraki-shi**
**Osaka 567-8680 (JP)**
• **HANATANI, Akinori**
**Ibaraki-shi**
**Osaka 567-8680 (JP)**
• **SAKAMOTO, Sachiko**
**Ibaraki-shi**
**Osaka 567-8680 (JP)**

• **MARUYAMA, Sumiyo**
**Ibaraki-shi**
**Osaka 567-8680 (JP)**
• **AKEMI, Hitoshi**
**Ibaraki-shi**
**Osaka 567-8680 (JP)**

(74) Representative: **Hoffmann Eitle**
**Patent- und Rechtsanwälte PartmbB**
**Arabellastraße 30**
**81925 München (DE)**

(56) References cited:
EP-A1- 0 900 565       EP-A1- 2 098 232
EP-A1- 2 098 235       WO-A1-02/090210
WO-A1-03/032960       WO-A1-2006/082728
WO-A1-2007/129427       WO-A1-2007/129712
WO-A1-2008/044336       WO-A1-2008/066184
WO-A1-2008/066185       WO-A1-2008/066185
WO-A1-2008/166184       WO-A2-2008/021113
JP-A- 2007 217 328

EP 2 279 739 B2

**Description**

[Field of the Invention]

The present invention relates to a package comprising an adhesive preparation containing donepezil.

[Background of the Invention]

Donepezil is a basic drug that has acetyl cholinesterase inhibitory action and is used as an anti-Alzheimer's dementia drug. The majority of Alzheimer's dementia patients are elderly and there are many cases in which elderly patients have difficulty in swallowing oral preparations. In addition, there are also cases in which it may be difficult to take oral preparations for patients in which symptoms of Alzheimer's dementia have advanced. In such cases, transcutaneous, parenteral administration of donepezil is useful.

Adhesive preparations containing donepezil, having an adhesive layer containing donepezil on a support, are disclosed in, for example, Japanese Patent Application Laid-open No. H11-315016 (Patent Document 1), WO 2003/032960 (Patent Document 2) and WO 2006/082728 (Patent Document 3) as examples of realizing such transcutaneous, parenteral administration of donepezil.

However, according to findings of the inventors of the present invention, the above-mentioned adhesive preparations containing donepezil of the prior art were found to undergo discoloration of the adhesive layer (such as changes in hue, saturation or value) during long-term storage. Even if such discoloration of the adhesive layer consists of only a slight change and does not have any detrimental affect whatsoever on the efficacy of the adhesive preparation, it ends up instilling a sense of deteriorated quality among patients and causes problems such as making them hesitate to use it. Moreover, the adhesive preparations containing donepezil of the prior art as described above normally visually have a white to light yellow color (light brown color) when in the fresh state, which tends to make discoloration of the adhesive layer easily recognized extremely sensitively by patients. Consequently, although there has been a need to establish a technology for inhibiting discoloration over time of these adhesive layers containing donepezil, the mechanism thereof has yet to be adequately elucidated.

On the other hand, in the technical field of adhesive preparations, discoloration of the adhesive layer is known to occur during long-term storage, and technologies for inhibiting this are known, such as those disclosed in Japanese Patent No. 3124069 (Patent Document 4), Japanese Patent Application Laid-open No. H11-047233 (Patent Document 5), Japanese Translation of PCT Application No. 2006-523637 (Patent Document 6), and Japanese Translation of PCT Application No. 2003-530442 (Patent Document 7). However, these publications do not make any suggestion of time-based discoloration of an adhesive preparation containing donepezil or suggest the application to an adhesive preparation containing donepezil.

On the other hand, Japanese Patent Application Laid-open No. H3-034923 (Patent Document 8) discloses an adhesive preparation containing estradiol in which the water content in the adhesive layer of the preparation is made to be 1.0% by weight or less for the purpose of enhancing transcutaneous absorption of estradiol and long-term stability. Moreover, WO 1998/030210 (Patent Document 9) discloses an isosorbide nitrate adhesive preparation in which the water content of an adhesive composition is made to be 0.5% by weight or less for the purpose of enhancing transcutaneous absorption of isosorbide nitrate. However, neither of these publications make any suggestion whatsoever of time-based discoloration of an adhesive preparation containing donepezil or suggest application to an adhesive preparation containing donepezil.

[Patent Document 1] Japanese Patent Application Laid-open No. H11-315016
[Patent Document 2] WO 2003/032960
[Patent Document 3] WO 2006/082728
[Patent Document 4] Japanese Patent No. 3124069
[Patent Document 5] Japanese Patent Application Laid-open No. H11-047233
[Patent Document 6] Japanese Translation of PCT Application No. 2006-523637
[Patent Document 7] Japanese Translation of PCT Application No. 2003-530422
[Patent Document 8] Japanese Patent Application Laid-open No. H3-034923
[Patent Document 9] WO 1998/030210

[Disclosure of the Invention]

[Problems To Be Solved by the Invention]

An object of the present invention is to provide a package in which an adhesive preparation containing donepezil is housed, which inhibits discoloration over time of an adhesive layer. In addition, an object of the present invention is to provide a package in which an adhesive preparation containing donepezil is housed, which has superior releasability of donepezil from the adhesive layer.

[Means for Solving the Problems]

As a result of conducting extensive studies on the mechanism of discoloration over time in the adhesive layer of an adhesive preparation containing donepezil, the inventors of the present invention found that, in an adhesive preparation containing an acrylic adhesive and donepezil, there is a significant correlation between discoloration over time and the water content of the adhesive layer, and that discoloration over time of the adhesive layer can be inhibited and deterioration of releasability of donepezil from the adhesive layer can be inhibited by suitably controlling the water content of the adhesive layer, thereby leading to completion of the present invention.
Namely, the present invention provides the following inventions (1) and (2):

(1) a package, comprising: an adhesive preparation containing donepezil, having a support and an adhesive layer on at least one side of the support, the adhesive layer containing donepezil and an acrylic adhesive; and a moisture permeation preventing pouch, wherein the adhesive preparation is housed in the moisture permeation preventing pouch and a water content of the adhesive layer is controlled to be 2000 ppm or more and 5000 ppm or less;
(2) the package described in (1) above, wherein the adhesive preparation and a desiccant are housed in the moisture permeation preventing pouch.

In addition, the present invention also provides the following invention (3):

(3) a package comprising: an adhesive preparation containing donepezil, having a support and an adhesive layer on at least one side of the support, the adhesive layer containing donepezil and an acrylic adhesive; a moisture permeation preventing pouch; and a desiccant, wherein the adhesive preparation containing donepezil and the desiccant are housed in the moisture permeation preventing pouch, and a water content of the adhesive layer is 2000 ppm or more and 5000 ppm or less;

In addition, the present disclosure also provides the following aspects (4) to (5):

(4) a method for inhibiting discoloration over time of an adhesive layer, comprising: controlling a water content of the adhesive layer of an adhesive preparation containing donepezil, having an adhesive layer containing donepezil and an acrylic adhesive on at least one side of a support, to be 2000 ppm or more and 5000 ppm or less;
(5) a method for inhibiting deterioration of releasability of donepezil from an adhesive layer, comprising: controlling a water content of the adhesive layer of an adhesive preparation containing donepezil, having an adhesive layer containing donepezil and an acrylic adhesive on at least one side of a support, to be 2000 ppm or more and 5000 ppm or less.

[Advantageous Effects of the Invention]

According to the present invention, since discoloration over time of an adhesive layer can be inhibited, a package comprising an adhesive preparation containing donepezil can be realized, wherein the package and the adhesive preparation housed therein have enhanced stability over time and reliability. In addition, in a more preferable aspect, releasability of donepezil from the adhesive layer is superior and deterioration thereof over time can be inhibited. Thus, drug availability is effectively enhanced and as a result, quality of life (QOL) and economy are also enhanced.

[Description of the Embodiments for Carrying Out the Invention]

The following provides an explanation of the invention using preferred embodiments thereof.
The adhesive preparation containing donepezil housed in the package of the present invention has a support and an adhesive layer formed on at least one side of the support.

(Support)

Although there are no particular limitations on the material or form of the support provided it is able to stably support the adhesive layer, that which does not allow donepezil in the adhesive layer to permeate there through resulting in a decrease in the incorporated percentage thereof (namely, a material that is impermeable to donepezil) is preferable.

Specific examples of the support include, but are not limited to, single films made of materials such as polyester (such as polyethylene terephthalate (PET)), nylon, polyvinyl chloride, polyethylene, polypropylene, ethylene-vinyl acetate copolymer, polytetrafluoroethylene or ionomer resin, metal foils and laminated films in which one type or two or more types of these single films are laminated.

A porous film or a laminate of a porous film and another film or metal foil may also be used as a support from the viewpoint improving anchoring (adhesiveness) with the adhesive layer. Specific examples of porous films include paper, woven fabric, non-woven fabric (such as polyester non-woven fabric or polyethylene terephthalate non-woven fabric), single films made of polyester, nylon, Saran (product name), polyethylene, polypropylene, ethylene-vinyl acetate copolymer, polyvinyl chloride, ethylene-ethyl acrylate copolymer or polytetrafluoroethylene, metal foil or polyethylene terephthalate, and laminated films in which one type or two or more types of these single films are laminated, that have been subjected to mechanical perforation treatment. Among these, paper, woven fabric and non-woven fabric (such as polyester non-woven fabric or polyethylene terephthalate non-woven fabric) are preferable from the viewpoint of imparting flexibility to the support. In the case of employing such a porous film, although there are no particular limitations on the thickness thereof, normally the thickness is preferably about 10 to 500 $\mu$m, and in the case of thin adhesive preparations in the manner of plaster or adhesive tape, the thickness is preferably about 1 to 200 $\mu$m. In addition, in the case of using a woven fabric or non-woven fabric, the basis weight thereof is preferably made to be 5 to 30 g/m$^2$ from the viewpoint of improving anchoring strength.

Although there are no particular limitations thereon, the total thickness of the support is preferably 2 to 200 $\mu$m and more preferably 10 to 50 $\mu$m. If the total thickness is less than 2 $\mu$m, handling ease such as self-supportability tends to decrease, while if the total thickness exceeds 200 $\mu$m, flexibility and shape conformability decrease, tending to a cause of a sense of incongruity (stiff feel) during use.

In order to improve anchoring between the support and the adhesive layer, it is preferable to compose the support from a laminated film consisting of a non-porous film and porous film of the above-mentioned materials, and form the adhesive layer on the side of the porous film.

(Adhesive Layer)

The adhesive layer at least contains donepezil and an acrylic adhesive. The adhesive layer is at least provided on one side of the above-mentioned support, and may also be provided, for example, on both sides of the support.

Here, the term donepezil means the concept which encompasses not only ($\pm$)-2-[(1-benzylpiperidin-4-yl) methyl]-5,6-dimethoxyindan-1-one (free form), but also pharmacologically acceptable salts and esters thereof. Namely, the donepezil contained in the adhesive layer may be donepezil (free form), a pharmacologically acceptable salt thereof, or an ester thereof. The adhesive layer preferably contains donepezil (free form) from the viewpoint of enhancing transcutaneous absorption. Furthermore, in the following descriptions, donepezil and/or donepezil hydrochloride are collectively referred to as "donepezil" unless specifically indicated otherwise.

Although there are no particular limitations on the content of donepezil in the adhesive layer, it is preferably within the range of 1 to 30% by weight and more preferably within the range of 3 to 20% by weight based on the total weight of the adhesive layer. If the content of donepezil is less than 1% by weight, an amount of donepezil effective for treatment cannot be expected to be released, while if the content of donepezil exceeds 30% by weight, in addition to therapeutic effects reaching their limit, this also tends to be economically disadvantageous.

The acrylic adhesive contained in the adhesive layer means that which contains (meth)acrylic acid alkyl ester. Here, "(meth)acrylic" means both acrylic and methacrylic. The acrylic adhesive is preferably a copolymer that is copolymerized by having as an essential component thereof a monomer having a polar group (such as a carboxyl group), e.g. (meth)acrylic acid, and more preferably has for a principal component thereof (principal constituent unit) a (meth)acrylic acid alkyl ester.

From the viewpoints of ease of crosslinking treatment, adhesion to human skin or drug solubility and the like, the acrylic adhesive is preferably a copolymer of a (meth)acrylic acid alkyl ester (first monomer component) and a vinyl monomer having a functional group capable of being involved in a crosslinking reaction (second monomer component), or a copolymer obtained by further copolymerizing this polymer with another monomer (third monomer component). When composed in this manner, adhesion properties, drug solubility and the like can be controlled according to the types and ratios of the copolymerized monomers, thereby making it possible to enhance the degree of freedom of design.

Specific examples of the (meth)acrylic acid alkyl ester (first monomer component) include (meth)acrylic acid alkyl esters in which the alkyl group is a linear, branched or cyclic alkyl group having 1 to 18 carbon atoms (such as a methyl, ethyl, propyl, butyl, pentyl, hexyl, cyclohexyl, heptyl, octyl, 2-ethylhexyl, nonyl, decyl, undecyl, dodecyl or tridecyl group). Among these, (meth)acrylic acid alkyl esters in which the alkyl group is a linear, branched or cyclic alkyl group having 4 to 18 carbon atoms (such as a butyl, pentyl, hexyl, cyclohexyl, heptyl, octyl, 2-ethylhexyl, nonyl, decyl, undecyl or tridecyl group) are preferable. In addition, the use of a monomer component that lowers the glass transition temperature of the polymer is more preferable for imparting adhesiveness at room temperature, and examples of such monomer components include (meth)acrylic acid alkyl esters in which the alkyl group is a linear, branched or cyclic alkyl group having 4 to 8 carbon atoms (such as a butyl, pentyl, hexyl, cyclohexyl, heptyl, octyl or 2-ethylhexyl group, more preferably a butyl, 2-ethylhexyl or cyclohexyl group, and particularly preferably a 2-ethylhexyl group). Among these, butyl acrylate, 2-ethylhexyl acrylate, 2-ethylhexyl methacrylate, cyclohexyl acrylate and cyclohexyl methacrylate are particularly preferable, while 2-ethylhexyl acrylate is most preferable. Furthermore, one type of the first monomer component can be used alone or two or more types can be used in combination.

In the above-mentioned vinyl monomer having a functional group capable of being involved in a crosslinking reaction (second monomer component), examples of functional groups capable of being involved in a crosslinking reaction include a hydroxyl group, carboxyl group and vinyl group, and among these, hydroxyl groups and carboxyl groups are preferable. Specific examples of the second monomer component include (meth)acrylic acid hydroxyethyl ester, (meth)acrylic acid hydroxypropyl ester, (meth)acrylic acid, itaconic acid, maleic acid, maleic anhydride, mesaconic acid, citraconic acid and glutaconic acid. Among these, acrylic acid, methacrylic acid, acrylic acid hydroxyethyl ester (and particularly, acrylic acid 2-hydroxyethyl ester) are preferable from the viewpoint of availability, and acrylic acid is most preferable. Furthermore, one type of the second monomer component can be used alone or two or more types can be used in combination.

The above-mentioned other monomer (third monomer component) is mainly used to adjust cohesive force of the adhesive layer or adjust the solubility and/or releasability of donepezil. Specific examples of this third monomer component include vinyl esters such as vinyl acetate or vinyl propionate, vinyl ethers such as methyl vinyl ether or ethyl vinyl ether, vinyl amides such as N-vinyl-2-pyrrolidone or N-vinylcaprolactam, (meth)acrylic acid alkoxy esters such as (meth)acrylic acid methoxyethyl ester, (meth)acrylic acid ethoxyethyl ester or (meth)acrylic acid tetrahydrofurfuryl ester, hydroxyl group-containing monomers such as hydroxypropyl (meth)acrylate or $\alpha$-hydroxymethyl acrylate (which does not serve as a crosslinking site since it is used as a third monomer component), (meth)acrylic acid derivatives having an amide group such as (meth)acrylamide, dimethyl(meth)acrylamide, N-butyl(meth)acrylamide or N-methylol(meth)acrylamide, (meth)acrylic acid amino alkyl esters such as (meth)acrylic acid aminoethyl ester, (meth)acrylic acid dimethylaminoethyl ester or (meth)acrylic acid t-butylaminoethyl ester, (meth)acrylic acid alkoxy alkylene glycol esters such as (meth)acrylic acid methoxyethylene glycol ester, (meth)acrylic acid methoxydiethylene glycol ester, (meth)acrylic acid methoxypolyethylene glycol ester or (meth)acrylic acid methoxypolypropylene glycol ester, (meth)acrylonitriles, monomers having sulfonic acid such as styrene sulfonic acid, allyl sulfonic acid, sulfopropyl (meth)acrylate, (meth)acryloyloxynaphthalene sulfonic acid or acrylamide methyl sulfonic acid, and vinyl group-containing monomers such as vinyl piperidone, vinyl pyrimidine, vinyl piperazine, vinyl pyrrole, vinyl imidazole, vinyl oxazole or vinyl morpholine. Among these, vinyl esters and vinyl amides are preferable, and more specifically, vinyl acetate is preferable as a vinyl ester and N-vinyl-2-pyrrolidone is preferable as a vinyl amide. Furthermore, one type of this third monomer component can be used alone or two or more types can be used in combination.

Among these, the acrylic adhesive is preferably an acrylic acid 2-ethylhexyl ester/acrylic acid/N-vinyl-2-pyrrolidone copolymer, acrylic acid-2-ethylhexyl ester/acrylic acid 2-hydroxyethyl ester/vinyl acetate copolymer, or acrylic acid 2-ethylhexyl ester/acrylic acid copolymer, and more preferably an acrylic acid 2-ethylhexyl ester/acrylic acid/N-vinyl-2-pyrrolidone copolymer.

In the case the acrylic adhesive is a copolymer of a (meth)acrylic acid alkyl ester (first monomer component) and a vinyl monomer having a functional group capable of being involved in a crosslinking reaction (second monomer component), the weight ratio of the (meth)acrylic acid alkyl ester (first monomer component) to the vinyl monomer having a functional group capable of being involved in a crosslinking reaction (second monomer component) of the copolymer is preferably 99 to 85:1 to 15, and more preferably 99 to 90:1 to 10.

In addition, in the case the acrylic adhesive is a copolymer of a (meth)acrylic acid alkyl ester (first monomer component), a vinyl monomer having a functional group capable of being involved in a crosslinking reaction (second monomer component), and another monomer (third monomer component), the weight ratio of the (meth)acrylic acid alkyl ester (first monomer component) to the vinyl monomer having a functional group capable of being involved in a crosslinking reaction (second monomer component) and the other monomer (third monomer component) is preferably 40 to 94:1 to 15:5 to 50 and more preferably 50 to 89:1 to 10:10 to 40.

Although varying according to the polymer composition, normally the glass transition temperature of the acrylic adhesive is preferably -100 to -10°C and preferably -90 to -20°C from the viewpoint of adhesiveness of the adhesive preparation.

Although there are no particular limitations on the content of the acrylic adhesive in the adhesive layer, it is preferably within the range of 10 to 90% by weight, more preferably within the range of 20 to 80% by weight, and even more preferably within the range of 30 to 70% by weight based on the total weight of the adhesive layer.

The acrylic adhesive can be produced by suitably employing a conventionally known technique in accordance with ordinary methods. Although there are no particular limitations on the method used to polymerize the acrylic adhesive, an example of a polymerization method consists of adding a polymerization initiator (such as benzoyl peroxide or azobisisobutyronitrile) to each of the above-mentioned monomers in a solvent (such as ethyl acetate), and then allowing to react for 5 to 48 hours at 50 to 70°C.

The adhesive layer may also contain other adhesives in addition to the above-mentioned acrylic adhesive. Examples of other adhesives include, but are not limited to, rubber-based adhesives such as silicone rubber, polyisoprene rubber, polyisobutylene rubber, styrene-butadiene rubber, styrene-isoprene-styrene block copolymer rubber or styrene-butadiene-styrene block copolymer rubber, silicone-based adhesives, and vinyl-based polymer adhesives such as polyvinyl alcohol, polyvinyl alkyl ethers or polyvinyl acetate. Preferable examples of rubber-based adhesives include polyisobutylene and styrene-diene-styrene block copolymers (such as styrene-butadiene-styrene block copolymer (SBS) or styrene-isoprene-styrene block copolymer (SIS)). One type of these other adhesives can be used alone or two or more types can be used in combination.

The adhesive layer may also contain a liquid component from the viewpoints of imparting a soft feel during use, and reducing pain and skin irritation attributable to skin adhesive strength when peeling from the skin. A liquid component may be incorporated for the purpose of improving transcutaneous absorption and storage stability of donepezil or for the purpose of enhancing solubility of donepezil in the adhesive. Although there are no particular limitations on the liquid component provided it is a liquid at room temperature, it preferably demonstrates a plasticizing action and is compatible with the above-mentioned adhesive polymer that composes the adhesive.

The liquid component is preferably an organic liquid component from the viewpoint of compatibility with the adhesive layer. Specific examples of organic liquid components include fatty acid alkyl esters (such as esters of lower primary alcohols having 1 to 4 carbon atoms and saturated or unsaturated fatty acids having 12 to 16 carbon atoms), saturated or unsaturated fatty acids having 8 to 10 carbon atoms (such as caprylic acid (octanoic acid, C8), pelargonic acid (nonanoic acid, C9), capric acid (decanoic acid, C10) or lauric acid (C12)), glycols such as ethylene glycol, diethylene glycol, triethylene glycol, polyethylene glycol, propylene glycol or polypropylene glycol, oils such as olive oil, castor oil, squalene or lanolin, organic solvents such as ethyl acetate, ethyl alcohol, dimethyldecylsulfoxide, decylmethylsulfoxide, dimethylsulfoxide, dimethylformamide, dimethylacetoamide, dimethyllaurylamide, dodecylpyrrolidone, isosorbitol or oleyl alcohol, liquid surfactants, plasticizers such as diisopropyl adipate, phthalic acid esters or diethyl sebacate, and hydrocarbons such as liquid paraffin. In addition, other examples include ethoxylated stearyl alcohol, glycerin esters (those that are liquid at room temperature), isotridecyl myristate, N-methylpyrrolidone, ethyl oleate, oleic acid, diisopropyl adipate, octyl palmitate, 1,3-propanediol and glycerin. Among these, from the viewpoint of preparation stability and the like, fatty acid alkyl esters, saturated fatty acids, hydrocarbons and organic solvents are preferable, while fatty acid alkyl esters are more preferable. One type of these organic liquid components can be used alone or two or more types can be used in combination.

From the viewpoint of compatibility with the acrylic adhesive and the like, the organic liquid component is preferably a fatty acid alkyl ester and more preferably an ester of a lower primary alcohol having 1 to 4 carbon atoms and a saturated or unsaturated fatty acid having 12 to 16 carbon atoms. Here, the saturated or unsaturated fatty acid having 12 to 16 carbon atoms is preferably a saturated fatty acid, and the lower primary alcohol having 1 to 4 carbon atoms may be linear or branched. Preferable examples of fatty acids having 12 to 16 carbon atoms include lauric acid (C12), myristic acid (C14) and palmitic acid (C16), while preferable examples of lower primary alcohols having 1 to 4 carbon atoms include isopropyl alcohol, ethyl alcohol, methyl alcohol and propyl alcohol. Particularly preferable examples of fatty acid alkyl esters include isopropyl myristate, ethyl laurate and isopropyl palmitate.

Furthermore, in the case of using a fatty acid alkyl ester, a fatty acid having 8 to 10 carbon atoms and/or glycerin may be used in combination with the fatty acid alkyl ester from the viewpoint of improving transcutaneous absorption of donepezil.

Although there are no particular limitations on the content of the liquid component in the adhesive layer, it is preferably 10 to 80% by weight, more preferably 20 to 70% by weight, and even more preferably 30 to 60% by weight based on the total weight of the adhesive layer. If the incorporated amount is less than 10% by weight, a satisfactory soft feel cannot be obtained due to insufficient plasticization of the adhesive layer, thereby preventing the obtaining of adequate skin irritation reduction effects, while conversely, if the incorporated amount exceeds 80% by weight, the liquid component is unable to be retained in the adhesive by the cohesive force of the adhesive, thereby causing the occurrence of blooming on the surface of the adhesive layer and causing adhesive strength to become excessively weak, thereby increasing the possibility of the preparation coming off of the skin surface during use.

The adhesive layer may also contain a metal chloride. As a result of containing a metal chloride in the adhesive layer, decreases in cohesive force of the adhesive layer are reduced when the adhesive preparation is adhered to human skin, while also reducing the likelihood of cohesive failure when the adhesive layer is peeled off.

Specific examples of metal chlorides include, but are not limited to, chlorides of alkaline metals such as sodium or potassium, chlorides of alkaline earth metals such as calcium or magnesium, aluminum chloride, stannous chloride and ferric chloride. From the viewpoints of superior safety and ability to inhibit decreases in cohesive force of the adhesive layer, sodium chloride, calcium chloride, aluminum chloride, stannous chloride and ferric chloride are preferable, sodium chloride and calcium chloride are more preferable, and sodium chloride is particularly preferable. One type of these metal chlorides can be used alone or two or more types can be used in combination.

Although there are no particular limitations on the content of the metal chloride in the adhesive layer, it is preferably within the range of 0.1 to 20% by weight, more preferably 0.5 to 15% by weight, and most preferably 1 to 10% by weight based on the total weight of the adhesive layer. If the incorporated amount is less than 0.1% by weight, the effect of inhibiting decreases in cohesive force of the adhesive layer may be inadequate, while conversely, if the incorporated amount exceeds 20% by weight, although inhibitory effects are demonstrated, the metal chloride is unable to be uniformly dispersed in the adhesive (adhesive polymer), thereby causing the preparation to have a poor appearance.

The metal chloride may be formed in the adhesive layer formation process by neutralizing donepezil hydrochloride with an inorganic base containing a metal by mixing and stirring in a solvent. As a result, a drug-containing solution that contains a metal chloride can be prepared without having to add a metal chloride, and by using such a drug-containing solution containing a metal chloride, the metal chloride is made to be present with donepezil in the final adhesive layer. Examples of such inorganic bases containing a metal include, but are not limited to, inorganic bases of alkaline metals or alkaline earth metals, such as sodium hydroxide, potassium hydroxide, calcium hydroxide, magnesium hydroxide, sodium bicarbonate, potassium bicarbonate, sodium carbonate or potassium carbonate. From the viewpoint of reducing the likelihood of formation of by-products, hydroxides of alkaline metals or alkaline earth metals are preferable, sodium hydroxide, calcium hydroxide and magnesium hydroxide are more preferable, and sodium hydroxide is particularly preferable. Furthermore, after having formed a metal chloride by neutralizing donepezil hydrochloride with an inorganic base containing a metal, a metal chloride may be further added to the resulting drug (donepezil)-containing solution.

The adhesive layer may also contain a crosslinking agent as necessary. Specific examples of crosslinking agents include, but are not limited to, peroxides (such as benzoyl peroxide (BPO), metal oxides (such as magnesium aluminometasilicate), polyfunctional isocyanate compounds, organic metal compounds (such as zirconium alaninate, zinc alaninate, zinc acetate, zinc ammonium glycinate or titanium compounds), metal alcolates (such as tetraethyl titanate, tetraisopropyl titanate, aluminum isopropylate or aluminum sec-butryrate), and metal chelate compounds (such as dipropoxybis(acetylacetonato)titanium, tetraoctylene glycol titanate, aluminum isopropylate, aluminum ethylacetoacetate diisopropylate, aluminum tris(ethylacetoacetate) or aluminum tris(acetylacetonate)). Among these, from the viewpoint of efficiently being able to form crosslinks in the presence of donepezil, peroxides, metal oxides, organic metal compounds, metal alcolates and metal chelate compounds are preferable, while metal alcolates and metal chelate compounds are more preferable. From the viewpoint of facilitating the obtaining of crosslinked structures having a suitable crosslinked density, metal chelate compounds such as aluminum ethylacetoacetate diisopropylate are particularly preferable. One type of these crosslinking agents can be used alone or two or more types can be used in combination.

Although there are no particular limitations on the content of the crosslinking agent in the adhesive layer and the incorporated amount thereof varies according to the types of crosslinking agent and adhesive used, in general, the incorporated amount is preferably 0.1 to 1.0 parts by weight, more preferably 0.1 to 0.6 parts by weight, and even more preferably 0.2 to 0.5 parts by weight, based on 100 parts by weight of the adhesive. If the incorporated amount is less than 0.1 parts by weight, the number of crosslinking sites is excessively low and adequate cohesive force is unable to be imparted to the adhesive layer, thereby resulting in the risk of the occurrence of residual adhesive and strong skin irritation attributable to cohesive failure when peeling from the skin, while if the incorporated amount exceeds 1.0 parts by weight, although cohesive force is sufficiently large, adequate skin adhesive strength may be unable to be obtained, and there is also the risk of skin irritation due to the presence of residual, unreacted crosslinking agent.

The adhesive layer may also contain additives such as antioxidants, various types of pigments, various types of fillers, stabilizers, drug solubilization assistants or drug solubilization inhibitors as necessary. Examples of such stabilizers or antioxidants include, but are not limited to, ascorbic acid, metal salts or esters thereof, isoascorbic acid or metal salts thereof, ethylenediamine tetraacetate or metal salts thereof, cysteine, acetylcysteine, 2-mercaptobenzimidazole, 3(2)-t-butyl-4-hydroxyanisole, 2,6-di-t-butyl-4-methylphenol, pentaerythritol tetraquis[3-(3',5,'-di-t-butyl-4'-hydroxyphenyl)propionate], 3-mercapto-1,2-propanediol, tocopherol, tocopherol acetate, lutin, quercetin, hydroquinone, metal hydroxymethanesulfinates, hypophosphorous acid, metal metabisulfites, sodium bisulfite, metal sulfites and metal thiosulfates. Furthermore, one type of these compounds can be used alone or two or more types can be used in combination. The total content of these stabilizers or antioxidants is preferably within the range of 0.0005 to 5% by weight, more preferably within the range of 0.001 to 3% by weight, and even more preferably within the range of 0.01 to 1% by weight based on the total weight of the adhesive layer.

Although there are no particular limitations on the thickness of the adhesive layer, it is preferably 20 to 300 $\mu$m, more preferably 30 to 300 $\mu$m and even more preferably 50 to 300 $\mu$m. If the thickness of the adhesive layer is less than 20 $\mu$m, it tends to be difficult to obtain adequate adhesive strength and contain an effective amount of donepezil, while if the thickness of the adhesive layer exceeds 300 μm, it tends to be difficult to apply the adhesive layer.

The adhesive layer has a water content of 2000 ppm or more and 5000 ppm or less in order to inhibit discoloration over time and enhance stability and reliability over time. Here, the water content of the adhesive layer means the weight ratio of water contained in the adhesive layer (ratio of the weight of water to the total weight of the adhesive layer) as measured according to the Karl Fischer coulometric titration method, and in the present description, means the value measured under the measurement conditions described in the subsequent examples.

An adhesive layer containing an acrylic adhesive as the principal component thereof is adopted in the adhesive preparation containing donepezil housed in the package of the present invention, and the acrylic adhesive contained in this adhesive preparation has the role of making it easy to control the water content of the adhesive layer to 2000 ppm or more and 5000 ppm or less. Although the reason for this is not certain, it is surmised that, since acrylic adhesives have a polar -COO- group in their basic structure, they have a higher affinity for water as compared with, for example, adhesive layers having a rubber-based adhesive as a principal component thereof. According to findings of the inventors of the present invention, it was newly found that changes in the water content of such an adhesive layer are related to discoloration over time, and it was confirmed that discoloration of the adhesive layer over time could be inhibited by controlling the water content of the adhesive layer to 2000 ppm or more and 5000 ppm or less. In addition, although functional groups of the previously described vinyl monomer having a functional group capable of being involved in a crosslinking reaction (second monomer component) or other monomer (third monomer component) have the potential to cause discoloration of the adhesive layer as a result of reacting with other adhesive layer components and the like, an acrylic adhesive is particularly advantageous in terms of being able to easily inhibit such discoloration of the adhesive layer in the case of using an adhesive that contains such an acrylic adhesive.

Although definitive trends in releasability of donepezil from the adhesive layer have not been observed when the water content of the adhesive layer is within a certain range, a high-performance adhesive preparation having particularly high donepezil release rate was unexpectedly able to be realized by controlling the water content of the adhesive layer to 2000 ppm or more and 5000 ppm or less. In addition, although the reason for releasability of donepezil from the adhesive layer deteriorating over time is unclear, such deterioration over time was unexpectedly able to be inhibited and an adhesive preparation having superior releasability of donepezil even after long-term storage was able to be realized by controlling the water content of the adhesive layer to 2000 ppm or more and 5000 ppm or less.

Although there are no particular limitation on the method used to adjust the water content of the adhesive layer to within the above-mentioned ranges, a known method can be suitably selected for that purpose. Moisture content of the adhesive layer fluctuates dependent upon relative humidity of the external environment (atmosphere), and converges on the equilibrium water content at that temperature and humidity with the passage of time. Namely, if the relative humidity of the external environment (atmosphere) is high, the water content of the adhesive layer rises as a result of the adhesive layer absorbing and/or adsorbing moisture. Conversely, if the relative humidity of the external environment (atmosphere) is low, in addition to adsorption of moisture into the adhesive layer being inhibited, moisture is released from the adhesive layer, thereby causing the water content of the adhesive layer to decrease. Thus, the water content of the adhesive layer can be controlled by adjusting the temperature and/or relative humidity of the external environment (atmosphere). Furthermore, adjustment of the temperature and/or relative humidity of the external environment (atmosphere) can be suitably carried out with known air-conditioning equipment.

For example, by maintaining the atmosphere in the punching step and packaging stage of the production process of an adhesive preparation containing donepezil to a temperature of 20 ± 10°C and relative humidity of 30 ± 10% RH, and holding in that environment for 30 minutes to 48 hours, the water content of the adhesive layer can be controlled to 2000 to 5000 ppm.

(Release Liner)

The adhesive preparation containing donepezil preferably has a release liner on the adhesive layer from the viewpoint of protective the adhesive side of the adhesive layer until immediately prior to use. Namely, the adhesive preparation containing donepezil is preferably composed such that an adhesive layer is laminated on at least one side of a support, and a release liner is laminated on the adhesive side of the adhesive layer (the side opposite from the side laminated to the support).

A known release liner can be suitably selected for use as the release liner. Specific examples of release liners include, but are not limited to, plastic films having high releasability, release liners in which a release layer composed of a release treatment agent is laminated on the surface of a release liner base material, and release liners in which a plastic film having high releasabiity as described above is laminated as a release layer on the surface of a release liner base material. The release layer can be formed by, for example, coating or laminating the above-mentioned release treatment agent or a material of the above-mentioned plastic film having high releasability onto the above-mentioned release liner base material. Furthermore, the release side of the release liner may be formed only on one side or on both sides of the release liner base material.

Examples of the release liner base material include, but are not limited to, plastic films such as polyethylene terephthalate (PET) film, polyimide film, polypropylene film, polyethylene film, polycarbonate film or polyester film (excluding PET), metal-deposited plastic films in which a metal is deposited on these plastic films, paper such as Japanese paper, regular paper, kraft paper, glassine paper or wood-free paper, base materials made from fibrous materials such as non-woven fabric or woven fabric, and metal foils.

There are no particular limitations on the release treatment agent, and examples include polymers containing long-chain alkyl groups, silicone polymers (silicone-based release agents) and fluorine-based polymers (fluorine-based release agents).

Specific examples of plastic films having high releasability *per se* include but are not limited to, polyethylene (including low-density polyethylene and linear low-density polyethylene), polypropylene, and ethylene-$\alpha$-olefin copolymers (block copolymers or random copolymers) including ethylene-propylene copolymers, as well as polyolefin-based films of polyolefin-based resins composed of mixtures thereof, and Teflon™ films.

Although there are no particular limitations on the total thickness of the release liner, normally the thickness is preferably 200 $\mu$m or less and more preferably 25 to 100 $\mu$m.

Furthermore, a backing treatment agent such as a silicone-based polymer, fluorine-based polymer or wax can be coated onto the back of the support of the adhesive preparation containing donepezil without using a release liner, and this can be wound into the shape of a roll.

(Production Method)

Although there are no particular limitations on the production method of the adhesive preparation containing donepezil, an example of simple method consists of preparing a mixture containing at least donepezil and an acrylic adhesive, and depositing this mixture by coating onto a support.

More specifically, an adhesive preparation containing donepezil can be produced by preparing a mixture (solution or dispersion) by dissolving or dispersing donepezil, an acrylic adhesive and as necessary, a metal chloride, stabilizer or crosslinking agent and the like, in a solvent, depositing the mixture obtained in this manner onto at least one side of a support by coating and drying thereon, and after thus forming an adhesive layer on the surface of the support, providing a protective release liner as previously described on the adhesive layer as necessary. In addition, an adhesive preparation containing donepezil can also be produced by forming the adhesive layer on the surface of the protective release liner by depositing the above mixture on at least one side of the release liner by coating and drying thereon, followed by adhering the support on the adhesive layer of the release liner.

Examples of solvents used when preparing the above-mentioned mixture include, but are not limited to, ethyl acetate, toluene, hexane, 2-propanol, methanol, ethanol and water. Furthermore, these solvents can also be used to adjust viscosity after having added a crosslinking agent.

Crosslinking treatment may also be carried out on the adhesive layer as necessary. The crosslinking treatment of the adhesive layer is known, examples of which include chemical crosslinking treatment (such as crosslinking treatment using a crosslinking agent) and physical crosslinking treatment (such as crosslinking treatment by irradiating with gamma rays or other electron beam or by irradiating with ultraviolet light), and can be carried out by a technique typically carried out in the art. Furthermore, crosslinking treatment of the adhesive layer is preferably chemical crosslinking treatment from the viewpoint of reducing the likelihood of having a detrimental effect on donepezil.

In the case of carrying out chemical crosslinking treatment using a crosslinking agent, a functional group capable of being involved in a crosslinking reaction, such as a hydroxyl group, carboxyl group or vinyl group, is preferably introduced into the adhesive layer. Introduction of a functional group capable of being involved in a crosslinking reaction can itself be carried out by a known method. For example, introduction into the acrylic adhesive can be carried out by adding a monomer having a hydroxyl group, such as hydroxyethyl (meth)acrylate, or adding a monomer having a carboxyl group, such as acrylic acid or maleic acid, and copolymerizing during synthesis of the acrylic adhesive. Furthermore, chemical crosslinking treatment of the adhesive layer may also be carried out by a method that causes intermolecular or intramolecular crosslinking during a polymerization reaction by adding a monomer having two or more vinyl groups, such as divinylbenzene or ethylene glycol dimethacrylate, and copolymerizing when synthesizing the acrylic adhesive.

In the case of carrying out chemical crosslinking treatment using a crosslinking agent, it is preferable to carry out a step in which the adhesive layer is heated and held at a temperature equal to higher than the temperature of the crosslinking reaction, namely aging treatment (aging step). The temperature conditions and treatment time at this time are suitably selected corresponding to the type of crosslinking agent and the like, and although there are no particular limitations thereon, aging is normally carried out for about 12 to 96 hours at about 60 to 90°C and preferably for about 24 to 72 hours at about 60 to 80°C.

(Adhesive Preparation Containing Donepezil)

The adhesive preparation containing donepezil housed in the package of the present invention can be used as an anti-Alzheimer's dementia drug. In addition, other applications include, but are not limited to, prevention of cerebrovascular dementia and migraine headaches.

Although varying according to patient age, body weight, symptoms and the like, the adult dosage and administration method of the adhesive preparation containing donepezil preferably consist of application of an adhesive preparation containing 2 to 150 mg of donepezil or donepezil hydrochloride to an area of the skin measuring 5 to 120 $cm^2$ for about 1 to 7 days.

Furthermore, there are no particular limitations on the form of the adhesive preparation containing donepezil provided it allows the preparation to be adhered to the skin. Namely, the concept of the adhesive preparation includes, for example, preparations in the form of a tape, sheet, matrix, reservoir or controlled-release film.

(Package Of Adhesive Preparation Containing Donepezil Housed Within)

The package of the present invention controls the water content of the adhesive layer to 2000 ppm or more and 5000 ppm or less by housing the above-mentioned adhesive preparation containing donepezil in a moisture permeation preventing pouch.

The moisture permeation preventing pouch maintains humidity within the pouch at a constant level by inhibiting permeation of moisture, and can be suitably selected for use from among such pouches commonly and widely used for such purpose. The following provides an explanation of a laminated film having a layer composition consisting of (A) a layer that imparts physical strength, (B) a layer that blocks moisture, and (C) a sealant layer as a specific example of a moisture permeation preventing pouch.

Specific examples of the layer that imparts physical strength (A) include layers composed of polyester, high-density polyethylene, polypropylene, polyamide, vinyl chloride. The thickness of the layer that imparts physical strength (A) is preferably about 10 to 100 $\mu$m.

Specific examples of the layer that blocks moisture (B) include metal foils such as aluminum foil. The thickness of the layer that blocks moisture (B) is preferably 5 to 90 $\mu$m.

Specific examples of the sealant layer (C) include layers composed of polyacrylonitrile resins such as methyl acrylate-butadiene-acrylonitrile copolymer, low-density polyethylene, ethylene-vinyl acetate copolymer and ethylene-vinyl alcohol copolymer. Since polyacrylonitrile resins are lowly adsorbent with respect to donepezil, they are effective for stabilizing the content of donepezil in the preparation. The thickness of the sealant layer (C) is preferably about 10 to 100 $\mu$m. This thickness is particularly preferable for maintaining a stable adhesive preparation containing donepezil.

In a preferable aspect of the moisture permeation preventing pouch, the layer that imparts physical strength (A), the layer that blocks moisture (B) and the sealant layer (C) constitute a laminated film consisting of a PET film, aluminum foil and a polyacrylonitrile film, and the use of aluminum foil having a thickness of 6 $\mu$m or more demonstrates the desired moisture-proofing effect. Housing an adhesive preparation containing donepezil by using such a moisture permeation preventing pouch with the polyacrylonitrile resin film located on the inside makes it possible to maintain the water content of the adhesive layer immediately after production at a nearly constant level even after long-term storage.

Housing an adhesive preparation containing donepezil, in which the water content of the adhesive layer has been adjusted to 2000 ppm or more and 5000 ppm or less in the above-mentioned moisture permeation preventing pouch enables the water content of the adhesive layer to controlled to a roughly constant level over a long period of time.

In addition, enclosing a desiccant such as magnesium sulfate, silica or zeolite in the moisture permeation preventing pouch makes it possible to control the water content of the adhesive layer to a lower level than that prior to housing. For example, by enclosing an adhesive preparation containing donepezil, in which the water content of the adhesive layer has been adjusted to 2000 ppm or more and 5000 ppm or less, in the moisture permeation preventing touch along with a desiccant enables the water content of the adhesive layer to be controlled to 2000 ppm or more and 5000 ppm or less. However, since discoloration of the adhesive layer can occur if the moisture content of the adhesive layer within the moisture permeation preventing pouch becomes excessively low, the amount of desiccant used is preferably suitably adjusted corresponding to the performance of that desiccant.

[Examples]

Although the following provides a more detailed explanation of the present invention through examples and comparative examples thereof, the present invention is not limited thereto, and can be suitably modified within a range that does not deviate from the gist thereof. Furthermore, in the following explanations, the terms "parts" and "percent (%)" refer to "parts by weight" and "percent (%) by weight", respectively, unless specifically indicated otherwise.

The following describes methods used to measure various physical properties in the examples and comparative examples along with an example of a method used to produce the coated laminate used in the examples and comparative examples.

<Donepezil Release Rate>

The release rate of donepezil (%; ratio of the weight of eluted donepezil to the total weight of donepezil contained in a test piece) was measured under the following conditions based on the Dissolution Test Method 2 (Paddle Method) of the General Tests, Processes and Apparatuses of the Japanese Pharmacopeia.

Test solution: pH 4.0, acetic acid/sodium acetate buffer solution (0.05 mol/L) (prepared by dissolving 54.44 g of sodium acetate trihydrate in 3000 mL of distilled water, adding acetic acid to adjust to pH 3.95 to 4.04, followed by bringing to a total volume of 8000 mL with distilled water)
Test solution volume: 900 mL
Temperature: 32°C
Paddle rotating speed: 50 rpm
Elution time: 4 hours
HPLC conditions:
Column: Inertsil™ ODS-2 (GL Science) (4.6 mm I.D. x 15 cm, 5 $\mu$m)
Mobile Phase: 10 mM aqueous sodium 1-decanesulfonate solution/acetonitrile/70% perchloric acid = 650/350/1 (volume ratio) (concentration of aqueous sodium 1-decanesulfonate solution was made to be 10 mM based on entire mobile phase)
Column temperature: 35°C
Detection wavelength: UV (271 nm)
Sample injection volume: 50 $\mu$L
Flow rate: 1.4 mL/min
Retention time: 11.0 min

<Adhesive Layer Water Content>

Unpackaged adhesive preparations containing donepezil immediately after production (controlled temperature and controlled humidity) were promptly placed in an environment controlled to a temperature of $23\pm2$°C and relative humidity of $40\pm5$% RH and then punched out to a size of 7.5 cm$^2$ to produce test pieces. Subsequently, the release liner was removed from the test pieces and the test pieces were placed in a moisture vaporization apparatus. Furthermore, this processing was completed within 1 minute from production (controlled temperature and controlled humidity).

On the other hand, packaged adhesive preparations containing donepezil were opened in an environment controlled to a temperature of $23\pm2$°C and relative humidity of $40\pm5$% RH, and the removed adhesive preparations containing donepezil were punched out to a size of 7.5 cm$^2$ to produce test pieces. Subsequently, the release liner was removed from the test pieces and the test pieces were placed in a moisture vaporization apparatus. Furthermore, this processing was completed within 1 minute from opening of the package.

The test pieces within the moisture vaporization apparatus were heated at 140°C, the moisture generated there from was introduced into a titration flask using nitrogen as a carrier, and the water content of the adhesive layer (ppm; ratio of the weight of water to the total weight of the adhesive layer) was measured according to the Karl Fischer coulometric titration method.

<Production Example of Coated Laminate>

First, 75 parts of 2-ethylhexyl acrylate, 22 parts of N-vinyl-2-pyrrolidone, 3 parts of acrylic acid and 0.2 parts of azobisisobutyronitrile were solution-polymerized in ethyl acetate at 60°C in an inactive gas atmosphere to obtain an ethyl acetate solution of an acrylic adhesive (adhesive solid fraction: 28%). Next, this acrylic adhesive was mixed with donepezil according to the composition indicated below followed by adjusting the viscosity with ethyl acetate to obtain a coating solution.

Composition

| | |
|---|---|
| Donepezil (free base) | 7.57 wt% |

(continued)

| | |
|---|---|
| Acrylic adhesive | 40.53 wt% |
| Isopropyl myristate | 50.00 wt% |
| Sodium chloride | 1.16 wt% |
| Sodium hydroxide | 0.01 wt% |
| Sodium metabisulfite | 0.50 wt% |
| Ascorbic acid | 0.05 wt% |
| Aluminum ethylacetoacetate diisopropylate | 0.18 wt% |
| | (Total: 100 wt%) |

After coating the resulting coating solution onto a polyethylene terephthalate (PET) release liner to a thickness after drying of 150 μm, the coating solution was dried to deposit an adhesive layer on the release liner. Next, the adhesive layer of the release liner was laminated onto the PET non-woven fabric side of a PET support composed of a laminated film consisting of a PET non-woven fabric and PET film, followed by storing for 48 hours at 70°C to obtain a coated laminate composed of three layers consisting of a PET film, adhesive layer and PET non-woven fabric-PET film.

Production Example 1 and Comparative Example 1

After allowing the above-mentioned coated laminate to stand for 3 hours or more in an environment controlled to 23°C and 30% RH, the coated laminate was punched out to a size of 40 cm$^2$ to produce the adhesive preparation containing donepezil of Example 1. In addition, the adhesive preparation containing donepezil of Comparative Example 1 was produced in the same manner as that of Example 1 with the exception of allowing to stand in an environment controlled to 23°C and 75% RH. The resulting adhesive preparations containing donepezil of Example 1 and Comparative Example 1 were measured for water content of the adhesive layer and donepezil release rate immediately after production. The results are shown in Table 1.

[Table 1]

| | Controlled Conditions °C, %RH | Water Content ppm | Release Rate % |
|---|---|---|---|
| Production Example 1 | 23°C, 30% RH | 3034 | 82.3 ○ |
| Comparative Example 1 | 23°C, 75% RH | 11117 | 77.9 × |

As shown in Table 1, it was confirmed that the water content of the adhesive layer was fluctuate according to the relative humidity of the external environment. In addition, it was confirmed that donepezil release rate was affected by the water content of the adhesive layer, and more specifically, it was confirmed that the donepezil release rate decreased as the water content of the adhesive layer increased.

Production Examples 2 and 3, Comparative Examples 2 to 5, and Reference Examples 4 and 5

Adhesive preparations containing donepezil of Production Examples 2 and 3 and Comparative Examples 2 to 5 and Reference Examples 4 and 5 were produced by humidifying the adhesive preparation containing donepezil of Production Example 1 for fixed periods of time in a constant temperature chamber set to 40°C and 75% RH. The resulting adhesive preparations containing donepezil of Production Examples 2 and 3, Comparative Examples 2 to 5 and Reference Examples 4 and 5 were measured for water content of the adhesive layer and donepezil release rate of the adhesive layer immediately after production. The results are shown in Table 2.

[Table 2]

| | Controlled Humidity Time hr | Water Content ppm | Release Rate % |
|---|---|---|---|
| Production Example 2 | 0 | 3272 | 82.3 ○ |
| Production Example 3 | 0 | 3219 | 82.6 ○ |
| Reference Example 4 | 18 | 7481 | 81.6 ○ |
| Reference Example 5 | 18 | 7768 | 81.7 ○ |

(continued)

|  | Controlled Humidity Time hr | Water Content ppm | Release Rate % |
|---|---|---|---|
| Comparative Example 2 | 36 | 10208 | 78.7 × |
| Comparative Example 3 | 36 | 9685 | 79.4 × |
| Comparative Example 4 | 45 | 10963 | 77.3 × |
| Comparative Example 5 | 45 | 10751 | 77.7 × |

As shown in Table 2, the donepezil release rates of the adhesive preparations containing donepezil of Production Examples 2 and 3, and Reference Examples 4 and 5 in which the water content of the adhesive layer was 8000 ppm or less, exceeded 80%, and were confirmed to demonstrate superior donepezil releasability in comparison with the adhesive preparations containing donepezil of Comparative Examples 2 to 5, in which the water content of the adhesive layer exceeded 8000 ppm.

Examples 6 and 7, Comparative Examples 6 to 9 and Reference Examples 8 and 9

The adhesive preparations containing donepezil of Production Examples 2 and 3, Comparative Examples 2 to 5 and Reference Examples 4 and 5 were packaged using a trilayer film consisting of a PET film (thickness: 12 $\mu$m), aluminum foil (thickness: 7 $\mu$m) and polyacrylonitrile resin film (thickness: 30 $\mu$m) with the polyacrylonitrile resin film layer located on the inside. The obtained packages were subjected to long-term storage (40°C, 2 months) to produce packages of Examples 6 and 7, Comparative Examples 6 to 9 and Reference Examples 8 and 9 housing the adhesive preparations containing donepezil in a moisture permeation preventing pouches. Subsequently, the resulting packages of Examples 6 and 7, Comparative Examples 6 to 9 and Reference Examples 8 and 9 were opened, the adhesive preparations containing donepezil were taken out, and the water content of the adhesive layer and donepezil release rate of the adhesive layer after long-term storage were measured. The results are shown in Table 3.

[Table 3]

|  | Controlled Humidity Time hr | Water Content ppm | Release Rate % |
|---|---|---|---|
| Example 6 | 0 | 3408 | 83.3 ○ |
| Example 7 | 0 | 3337 | 85.0 ○ |
| Reference Example 8 | 18 | 6840 | 80.6 ○ |
| Reference Example 9 | 18 | 6248 | 80.2 ○ |
| Comparative Example 6 | 36 | 7980 | 74.0 × |
| Comparative Example 7 | 36 | 7934 | 76.7 × |
| Comparative Example 8 | 45 | 8816 | 73.1 × |
| Comparative Example 9 | 45 | 9516 | 73.2 × |

As shown in Table 3, in each of the packages of Examples 6 and 7, Comparative Examples 6 to 9 and Reference Examples 8 and 9, the water content of the adhesive layer of the adhesive preparations containing donepezil were controlled to roughly constant levels (within a range of about ±20%).

In addition, the donepezil release rates after long-term storage of the adhesive preparations containing donepezil of Examples 6 and 7, and Reference Examples 8 and 9 in which the water content of the adhesive layer was 7000 ppm or less, exceeded 80%, and were confirmed to demonstrate superior donepezil releasability in comparison with the adhesive preparations containing donepezil of Comparative Examples 6 to 9, in which the water content of the adhesive layer exceeded 7000 ppm.

Moreover, it was confirmed that deterioration over time of donepezil releasability was inhibited in the adhesive preparations containing donepezil of Examples 6 and 7, and Reference Examples 8 and 9 in which the water content of the adhesive layer was 7000 ppm or less, as compared with the adhesive preparations containing donepezil of Comparative Examples 6 to 9.

Reference Example 1, Examples 10 and 11 and Comparative Example 10

A coated laminated as previously described was processed in the same manner as Production Example 1 to produce an adhesive preparation containing donepezil of Reference Example 1 having a water content of the adhesive layer of 3195 ppm.

Next, using the same trilayer laminated film as Example 6, the adhesive preparation containing donepezil of Reference Example 1 was housed (packaged) with 5 g of a magnesium sulfate desiccant therein followed by long-term storage (40°C, 1 month) to produce the package of Example 10 in which an adhesive preparation containing donepezil was contained in a moisture permeation preventing pouch. Subsequently, the resulting package of Example 10 was opened, the adhesive preparation containing donepezil was taken out, and the water content of the adhesive layer after long-term storage was determined to be 2580 ppm.

Next, the package of Example 11 was produced in the same manner as that of Example 10 with the exception of not using a desiccant. When the water content of the adhesive layer after long-term storage was measured in the same manner as Example 10, it was determined to be 3333 ppm.

Moreover, the package of Comparative Example 10 was produced in the same manner as that of Example 10 with the exception of using 5 g of zeolite for the desiccant. When the water content of the adhesive layer after long-term storage was measured in the same manner as Example 10, it was determined to be 415 ppm.

Next, the color values (L*, a*, b*) as represented by the CIE 1976 (L*a*b*) color coordinate system (L-star, a-star, b-star color coordinate system, JIS Z 8729) were measured for each of the adhesive preparations containing donepezil. Using the color values (L*s, a*s, b*s) of the adhesive preparation containing donepezil of Reference Example 1 as reference points, the color differences ($\Delta$E*ab1) with the color values (L*1, a*1, b*1) of the adhesive preparations containing donepezil of Example 10, Example 11 and Comparative Example 10 were calculated in accordance with the following formula 1. The results are shown in Table 4.

$$\Delta E*ab1 = \{(L*1-L*s)^2+(a*1-a*s)^2+(b*1-b*s)^2\}^{1/2} \qquad (1)$$

[Table 4]

| | Water content ppm | Color Values | | | Color Difference | | | $\Delta$E*ab1 |
|---|---|---|---|---|---|---|---|---|
| | | L* | a* | b* | $\Delta$L* | $\Delta$a* | $\Delta$b* | |
| Reference Example 1 | 3195 | 95.51 | -5.63 | 7.99 | -- | -- | -- | -- |
| Example 10 | 2580 | 95.39 | -5.65 | 8.08 | -0.12 | -0.02 | 0.09 | 0.2 |
| Example 11 | 3333 | 95.37 | -5.64 | 8.10 | -0.14 | -0.01 | 0.11 | 0.2 |
| Comparative Example 10 | 415 | 94.62 | -6.41 | 12.10 | -0.89 | -0.78 | 4.11 | 4.3 |

As shown in Table 4, the adhesive preparations containing donepezil of Examples 10 and 11, in which the water content of the adhesive layer was 1000 ppm or more, were able to realize a visual appearance having a nearly white color, and it was confirmed that discoloration over time was inhibited in comparison with the adhesive preparation containing donepezil of Comparative Example 10.

[Industrial Applicability]

Since the package of the present invention and the adhesive preparation containing donepezil housed therein have superior quality stability, stability over time, drug availability and economy, and are able to enhance quality of life (QOL), they can be widely and effectively used in pharmaceutical applications against Alzheimer's dementia, cerebrovascular dementia or the prevention of migraine headaches and the like by parenteral administration, and particularly transcutaneous administration.

**Claims**

1. A package comprising:

an adhesive preparation containing donepezil, having a support and an adhesive layer on at least one side of the support, the adhesive layer containing donepezil and an acrylic adhesive; and

a moisture permeation preventing pouch,

wherein the adhesive preparation is housed in the moisture permeation preventing pouch, and a water content of the adhesive layer is controlled to be 2000 ppm or more and 5000 ppm or less.

2. The package according to claim 1,

wherein the adhesive preparation and a desiccant are housed in the moisture permeation preventing pouch.

**Patentansprüche**

1. Verpackung, umfassend:

ein Klebstoffpräparat mit Donepezil, welches einen Träger und eine Klebstoffschicht auf mindestens einer Seite des Trägers aufweist, wobei die Klebstoffschicht Donepezil und einen Acrylklebstoff enthält; und

einen Beutel, der ein Eindringen von Feuchtigkeit verhindert,

wobei das Klebstoffpräparat im Beutel, der ein Eindringen von Feuchtigkeit verhindert, untergebracht ist und der Wassergehalt der Klebstoffschicht so gesteuert wird, dass er 2000 ppm oder mehr und 5000 ppm oder weniger beträgt.

2. Verpackung gemäß Anspruch 1, wobei das Klebstoffpräparat und ein Trockenmittel im Beutel, der ein Eindringen von Feuchtigkeit verhindert, untergebracht sind.

**Revendications**

1. Emballage comprenant :

une préparation adhésive contenant du donépézil, ayant un support et une couche adhésive sur au moins un côté du support, la couche adhésive contenant du donépézil et un adhésif acrylique ; et

un sachet empêchant la pénétration d'humidité,

dans lequel la préparation adhésive est logée dans le sachet empêchant la pénétration d'humidité, et une teneur en eau de la couche adhésive est contrôlée pour être de 2000 ppm ou plus et 5000 ppm ou moins.

2. L'emballage selon la revendication 1,

dans lequel la préparation adhésive et un déshydratant sont logés dans le sachet empêchant la pénétration d'humidité.

**EP 2 279 739 B2**

**REFERENCES CITED IN THE DESCRIPTION**

**Patent documents cited in the description**

- JP H11315016 B
- WO 2003032960 A
- WO 2006082728 A
- JP 3124069 B
- JP H11047233 B
- JP 2006523637 W
- JP 2003530442 W
- JP H3034923 B
- WO 1998030210 A
- JP 2003530422 W